(19) **Europäisches Patentamt / European Patent Office / Office européen des brevets**

(11) **EP 1 996 626 B1**

(12) **FASCICULE DE BREVET EUROPEEN**

(45) Date de publication et mention de la délivrance du brevet:
**26.10.2011 Bulletin 2011/43**

(51) Int Cl.:
*C07K 16/42* (2006.01)        *C12N 5/12* (2006.01)
*C12N 15/13* (2006.01)        *A61K 39/395* (2006.01)
*G01N 33/577* (2006.01)        *A61P 7/04* (2006.01)

(21) Numéro de dépôt: **07731046.4**

(22) Date de dépôt: **26.02.2007**

(86) Numéro de dépôt international:
**PCT/FR2007/000342**

(87) Numéro de publication internationale:
**WO 2007/096536 (30.08.2007 Gazette 2007/35)**

(54) **ANTICORPS ANTI-IDIOTYPIQUES NEUTRALISANT L'ACTIVITE INHIBITRICE D'UN ANTICORPS INHIBITEUR DIRIGE CONTRE LE DOMAINE C1 DU FACTEUR VIII**

ANTIIDIOTYPISCHER ANTIKÖRPER, DER DIE INHIBIERENDE WIRKUNG EINES GEGEN DIE C1-DOMÄNE VON FAKTOR VIII GERICHTETEN INHIBIERENDEN ANTIKÖRPERS NEUTRALISIERT

ANTI-IDIOTYPIC ANTIBODY WHICH NEUTRALIZES THE INHIBITORY ACTIVITY OF AN INHIBITORY ANTIBODY DIRECTED AGAINST THE C1 DOMAIN OF FACTOR VIII

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **24.02.2006 FR 0601633**

(43) Date de publication de la demande:
**03.12.2008 Bulletin 2008/49**

(60) Demande divisionnaire:
**09002329.2 / 2 100 903**

(73) Titulaire: **LFB Biotechnologies**
**91940 Les Ulis (FR)**

(72) Inventeurs:
• **GILLES, Jean-Guy**
  **B-1090 Bruxelles (BE)**
• **JACQUEMIN, Marc, G.**
  **B-5330 Sart-Bernard (BE)**
• **SAINT-REMY, Jean-Marie**
  **B-1390 Grez-Doiceau (BE)**
• **BEHRENS, Christian**
  **F-91120 Palaiseau (FR)**

(74) Mandataire: **Hirsch & Associés**
**58, avenue Marceau**
**75008 Paris (FR)**

(56) Documents cités:
**EP-A- 1 388 544        WO-A-01/04269**

WO-A2-2005/016455

• GILLES J G G ET AL: "ANTIBODIES TO IDIOTYPES OF HUMAN MONOCLONAL ANTI-FACTOR VIII (FVIII) ANTIBODIES NEUTRALISE THEIR INHIBITORY ACTIVITY" BLOOD, W.B.SAUNDERS COMPANY, ORLANDO, FL, US, vol. 94, no. SUPPL01,PART01, 15 novembre 1999 (1999-11-15), page 460A, XP001109476 ISSN: 0006-4971

• GILLES J G G ET AL: "IN VIVO RESTORATION OF NORMAL FACTOR VIII ACTIVITY BY ADMINISTRATION OF A MOUSE ANTIBODY DIRECTED TOWARDS IDIOTOPES OF HUMAN ANTI-C2 INHIBITORS" BLOOD, W.B.SAUNDERS COMPANY, ORLANDO, FL, US, vol. 100, no. 11, 16 novembre 2002 (2002-11-16), page ABSTRNO64, XP009066822 ISSN: 0006-4971

• KALLAS A ET AL: "EPITOPE SPECIFICITY OF ANTI-FVIII ANTIBODIES DURING IMMUNE TOLERANCE THERAPY WITH FACTOR VIII PREPARATION CONTAINING VON WILLEBRAND FACTOR" THROMBOSIS RESEARCH, TARRYTOWN, NY, US, vol. 107, no. 6, 15 septembre 2002 (2002-09-15), pages 291-302, XP008041463 ISSN: 0049-3848

- JACQUEMIN M ET AL: "A human antibody directed to the factor VIII C1 domain inhibits factor VIII cofactor activity and binding to von Willebrand factor" BLOOD, W.B.SAUNDERS COMPANY, ORLANDO, FL, US, vol. 95, no. 1, 1 janvier 2000 (2000-01-01), pages 156-163, XP002150704 ISSN: 0006-4971
- LAVIGNE-LISSALDE GERALDINE ET AL: "Anti-factor VIII antibodies - A 2005 update" THROMBOSIS AND HAEMOSTASIS, vol. 94, no. 4, octobre 2005 (2005-10), pages 760-769, XP002404354 ISSN: 0340-6245
- PIPE S W ET AL: "New high-technology products for the treatment of haemophilia." HAEMOPHILIA : THE OFFICIAL JOURNAL OF THE WORLD FEDERATION OF HEMOPHILIA. OCT 2004, vol. 10 Suppl 4, octobre 2004 (2004-10), pages 55-63, XP002404355 ISSN: 1351-8216

**Description**

**Art antérieur et introduction**

La présente invention se rapporte à un anticorps monoclonal anti-idiotypique dirigé contre un anticorps inhibiteur du facteur VIII se liant au domaine C1 du facteur VIII, ainsi qu'à une lignée cellulaire produisant cet anticorps monoclonal anti-idiotypique, à l'utilisation de cet anticorps monoclonal anti-idiotypique comme médicament, et plus particulièrement à son utilisation pour la fabrication d'un médicament destiné au traitement de l'hémophilie A.

L'hémophilie A est une affection héréditaire liée à une anomalie du chromosome X, qui se traduit par une incapacité à coaguler chez les personnes atteintes. Cette maladie est le résultat de mutations sur le gène d'une protéine intervenant dans la coagulation, le facteur VIII (FVIII), qui déterminent soit une absence totale du facteur VIII dans le sang, soit un déficit partiel. L'hémophilie A est la plus commune des déficiences affectant la coagulation sanguine : elle touche en France 1 homme sur 5000, ce qui représente 80% des patients atteints d'hémophilie. L'autre type d'hémophilie, l'hémophile B, touche 20% des patients atteints d'hémophilie ; elle est causée par une déficience en un autre facteur de coagulation, le facteur IX.

Le traitement actuel de l'hémophilie (de type A ou B) consiste à administrer par voie intraveineuse le facteur de coagulation déficient ou manquant. En France, le facteur VIII destiné au traitement des hémophiles est disponible sous forme de médicaments dérivés du sang fournis par le Laboratoire Français du Fractionnement et des Biotechnologies (LFB) ou des laboratoires pharmaceutiques internationaux, ainsi que sous forme de médicaments recombinants issus du génie génétique. En effet, l'ADN codant pour le facteur VIII a été isolé et exprimé dans des cellules de mammifères (Wood et al., Nature (1984) 312 : 330-337), et sa séquence en acides aminés déduite à partir de l'ADNc.

Le facteur VIII (FVIII) sécrété est une glycoprotéine de masse moléculaire de 300 Kda (2332 acides aminés) jouant un rôle clé dans l'activation de la voie intrinsèque de la coagulation. Le FVIII inactif est constitué de six domaines : A1 (résidus 1-372), A2 (résidus 373-740), B (résidus 741-1648), A3 (résidus 1649-2019), C1 (résidus 2020-2172), et C2 (résidus 2173-2332), de l'extrémité N-terminale à l'extrémité C-terminale. Après sécrétion, le FVIII interagit avec le facteur von Willebrand (vWF) qui le protège des protéases plasmatiques. Le FVIII se dissocie du vWF après clivage par la thrombine. Ce clivage aboutit à l'élimination du domaine B et à la formation d'un hétérodimère. C'est sous cette forme que le FVIII circule dans le plasma. Cet hétérodimère est constitué d'une chaîne lourde (A1, A2) et d'une chaîne légère (A3, C1, C2).

Lorsqu'il est perfusé à un patient hémophile, le facteur VIII se fixe sur le facteur von Willebrand dans la circulation sanguine du patient. Le facteur VIII activé agit comme cofacteur du facteur IX activé, accélérant la conversion du facteur X en facteur X activé. Le facteur X activé convertit la prothrombine en thrombine. La thrombine convertit alors le fibrinogène en fibrine et un caillot apparaît.

Le problème majeur rencontré lors de l'administration de facteur VIII est l'apparition chez le patient d'anticorps dirigés contre le facteur VIII, appelés « anticorps inhibiteurs ». Ces anticorps neutralisent l'activité procoagulante du facteur VIII, qui est rendu inactif dés qu'il est perfusé. Ainsi, le facteur de coagulation administré est détruit avant d'avoir pu enrayer l'hémorragie, ce qui constitue une complication grave de l'hémophilie, le traitement devenant inefficace. De plus, certains patients non hémophiles génétiquement peuvent développer des inhibiteurs contre le facteur VIII endogène : il s'agit d'une hémophilie acquise.

Des études ont montré que la réponse immune anti-facteur VIII est de type IgG polyclonale appartenant majoritairement à la sous-classe IgG4 et IgG1 et plus rarement IgG2. Les IgG3 ne sont jamais représentées. La chaîne légère est souvent de type Kappa. La surreprésentation des IgG4 est plus accentuée chez les hémophiles qui ont un inhibiteur installé depuis longtemps. Les domaines C2 et A2 de la molécule de FVIII sont les cibles privilégiées de la réponse immune bien que dans certains cas on détecte des anticorps dirigés contre le domaine A3. En passant le plasma de patients hémophiles au travers d'une colonne d'immunoadsorption sur laquelle est immobilisé du FVIII, il est possible de purifier les anticorps totaux anti-FVIII. Les quantités récoltées dépassent souvent 100μg par 10mg d'IgG totales (Gilles JG et al. (1993) Blood ; 82 : 2452-2461). Un modèle animal a été développé pour étudier la formation d'inhibiteurs du facteur VIII ; des rats immunisés avec du facteur VIII humain recombinant montrent une réponse immune rapide, de type polyclonale (Jarvis et al. Thromb Haemost. 1996 Feb;75(2):318-25). Les mécanismes par lesquels les anticorps anti-facteur VIII interfèrent avec la fonction du facteur VIII sont nombreux, et incluent l'interférence dans le clivage protéolytique du facteur VIII et dans l'interaction du facteur VIII avec différents partenaires comme le facteur Von Willebrand (vWF), les phospholipides (PL), le facteur IX, le facteur X activé (FXa) ou l'APC (Activated Protein C). Il existe plusieurs traitements permettant d'atténuer les conséquences de cette réponse immune, comme par exemple les traitements impliquant la desmopressine qui est une hormone synthétique stimulant la production de facteur VIII, les agents promoteurs de la coagulation comme les concentrés de complexes prothrombiques ou les concentrés de complexes prothrombiques activés, le facteur VIIa recombinant, la plasmaphérèse et les infusions de quantités importantes ou intermédiaires de facteur VIII. Toutefois, ces méthodes restent très coûteuses et peu efficaces.

Du fait de la complexité de l'analyse *in vivo* de cette réponse immune polyclonale, des équipes ont isolé des anticorps monoclonaux dirigés contre certains domaines du facteur VIII. Ainsi, un anticorps humain monoclonal de type IgG4kappa, LE2E9, a été isolé. Cet anticorps est dirigé contre le domaine C1 du facteur VIII et inhibe l'activité cofacteur du facteur VIII et sa liaison au facteur Von Willebrand (Jacquemin et al. (2000) Blood 95:156-163). De la même façon, un anticorps monoclonal humain dirigé contre le domaine C2 du facteur VIII, nommé BO2C11 (IgG4kappa), produit à partir d'un répertoire de cellules B mémoire d'un patient atteint d'hémophilie A avec inhibiteurs, a été isolé (Jacquemin et al. Blood 1998 Jul 15;92(2):496-506). BO2C11 reconnaît le domaine C2 du facteur VIII et inhibe sa liaison au facteur Von Willebrand et aux phospholipides. Il inhibe complètement l'activité procoagulante du facteur VIII natif et activé. Un autre exemple d'anticorps monoclonal est l'anticorps BOIIB2, dirigé contre le domaine A2 du facteur VIII. L'anticorps BOIIB2 inhibe à 99% l'activité du facteur VIII. En se liant au domaine A2, il peut interférer et inhiber la fixation du FIXa qui possède un site de fixation de faible affinité dans cette région du FVIII et dès lors inhiber l'activité enzymatique du FIXa. Le deuxième mode d'action envisageable est son interférence dans l'équilibre entre la forme hétéro-dimère (A2:A1 et A3:C1:C2) du FVIII et la forme hétéro-trimère (A2 et A1 et A3:C1:C2) du FVIII en accélérant la dissociation du domaine A2 de ces complexes, les rendant non fonctionnels. (Ananyeva NM et al (2004) Blood Coagul Fibrinolysis. Mar;15(2):109-24. Review).

Grâce à ces nouveaux outils, une autre stratégie de lutte contre les anticorps inhibiteurs du facteur VIII, plus récente, envisage l'administration d'anticorps anti-idiotypiques (anticorps ayant la capacité d'interagir avec la région variable d'autres anticorps) neutralisant les anticorps inhibiteurs (Saint-Rémy JM et al. (1999) Vox Sang 77 (suppl 1) : 21-24). Un anticorps anti-idiotypique murin, le 14C12, décrit dans le document WO 2004/014955, neutralise *in vivo* de façon dose-dépendante les propriétés inhibitrices de l'anticorps anti-facteur VIII cible (l'anticorps monoclonal BO2C11), qui est dirigé contre le domaine C2 du facteur VIII. La réponse immune anti-facteur VIII étant polyclonale, des anticorps anti-idiotypiques murins dirigés contre le domaine A2 du facteur VIII ont également été développés (et décrits dans le document FR 05 08320). Le domaine A2 est un domaine de 43 kD dont la fonction demeure peu connue, mais il a été démontré que des anticorps inhibiteurs dirigés contre le domaine A2 du facteur VIII inhibent la fonction du facteur VIIIa par inhibition de la conversion du complexe FXase/FX à l'état de transition (Lollar et al. J Clin Invest. 1994 Jun; 93(6): 2497-504, Fay et al. J Biol Chem. 1996 ; 271(11) : 6027-6032).

Toutefois, la réponse immune dirigée contre le facteur VIII est polyclonale, et par conséquent implique des anticorps inhibiteurs dirigés contre d'autres domaines que les domaines A2 et C2. En effet, si l'étude des spécificités épitopiques des anticorps anti-facteur VIII a révélé que la plupart des inhibiteurs reconnaît des zones restreintes de la molécule de facteur VIII situées sur le domaine A2 de la chaîne lourde et/ou sur le domaine C2 de la chaîne légère, d'autres épitopes sont parfois reconnus. En effet, certains plasmas de patients contiennent des anticorps capables de se lier au domaine C1 de la chaîne légère du facteur VIII (Moreau et al. 2000 ; 95(11) :3435-441 ; Jacquemin et al. 2000 ; 95(1) :156-162).

Ainsi, il reste un besoin d'autres outils permettant la neutralisation d'autres anticorps inhibiteurs du facteur VIII dirigés contre d'autres domaines du facteur VIII, afin de neutraliser plus complètement les réponses polyclonales anti-facteur VIII des patients hémophiles.

C'est pourquoi le Demandeur a cherché à mettre au point un nouvel outil de traitement de l'hémophilie A permettant la neutralisation d'anticorps inhibiteurs dirigés contre le domaine C1 du facteur VIII.

**Description détaillée de l'invention**

Un premier objet de l'invention se rapporte ainsi à un anticorps monoclonal anti-idiotypique tel que défini dans les revendications dirigé contre un anticorps humain inhibiteur du facteur VIII, l'anticorps inhibiteur étant dirigé contre le domaine C1 du facteur VIII.

Les séquences sont définies selon Kabat [Kabat et al., "Sequences of Proteins of Immunological Interest", NIH Publication, 91-3242 (1991)].

Par « anticorps inhibiteurs » ou « inhibiteurs » du facteur VIII, on entend désigner les anticorps qui inhibent tout ou partie de l'activité procoagulante du facteur VIII, notamment en s'y fixant, et particulièrement un anticorps anti-facteur VIII dont l'épitope est situé sur le facteur VIII. De manière avantageuse, l'anticorps selon l'invention possède la capacité de neutraliser au moins 20%, avantageusement au moins 30%, avantageusement au moins 40%, avantageusement au moins 50%, avantageusement au moins 60%, et de manière encore plus avantageuse au moins 70%, 80%, 90%, 99% ou 100% de l'activité inhibitrice de la coagulation des anticorps inhibiteurs dirigés contre le domaine C1 du facteur VIII, cibles des anticorps monoclonaux anti- idiotypiques selon l'invention. Cette capacité de neutralisation de l'activité inhibitrice de la coagulation des anticorps inhibiteurs peut être déterminée en mesurant l'activité du facteur VIII en présence d'un anticorps inhibiteur et d'un anticorps anti-idiotypique dans un test tel que le « factor VIII chromogen test » (Jacquemin et al. (1998) Blood 92, 494-506).

L'expression « anticorps anti-idiotypique » s'entend d'un anticorps dirigé contre la région variable des anticorps inhibiteurs cibles. Dans un aspect particulier de l'invention, l'anticorps anti-idiotypique selon l'invention est dirigé contre des anticorps inhibiteurs dont le domaine variable de la chaîne lourde s'apparente à la lignée germinale DP-10. De tels anticorps inhibiteurs peuvent être obtenus à partir d'êtres humains (par exemple du sérum de patients présentant des anticorps inhibiteurs) ou d'autres espèces animales comme la souris, le cheval, la chèvre, des primates non humains, cette liste n'étant pas limitative, par immunisation avec le facteur VIII ou des fragments dérivés du facteur VIII, et plus particulièrement avec un fragment comprenant tout ou partie du domaine C1. Avantageusement, l'anticorps inhibiteur cible de l'anticorps anti-idiotypique selon l'invention reconnaît le domaine C1 dans sa configuration native. Avantageusement, l'anticorps inhibiteur cible de l'anticorps anti-idiotypique selon l'invention ne reconnaît pas le même domaine avec une mutation R2150H.

L'anticorps monoclonal anti-idiotypique selon l'invention peut être d'origine humaine ou animale. De plus, il peut être obtenu de différentes manières. Par exemple, des cellules produisant des anticorps anti-idiotypiques peuvent être obtenues à partir de lymphocytes de sang périphérique de patients présentant des anticorps inhibiteurs anti-facteur VIII ou à partir d'individus sains. Ces cellules peuvent être immortalisées par des techniques bien connues de l'homme du métier et sélectionnées par rapport à la capacité des anticorps anti-idiotypiques produits de neutraliser les anticorps inhibiteurs dirigés contre le facteur VIII. Un autre moyen de produire l'anticorps anti-idiotypique monoclonal selon l'invention est l'immunisation d'animaux, avantageusement des souris, par injection d'anticorps inhibiteurs du facteur VIII dirigés contre le domaine C1 du facteur VIII, puis la fusion des lymphocytes de rate avec une lignée cellulaire de myélome, avantageusement de myélome de souris, suivie par l'identification et le clonage de cultures cellulaires produisant les anticorps anti-idiotypiques dirigés contre les anticorps inhibiteurs du facteur VIII.

Chaque région CDR de chacune des chaînes légères de l'anticorps anti-idiotypique décrit dans la présente demande possède une séquence peptidique ayant au moins 70% d'identité avec les séquences des régions CDR des chaînes légères de l'anticorps produit par l'hybridome 18B6 , et chaque région CDR de chacune de ses chaînes lourdes possède une séquence peptidique ayant au moins 70% d'identité avec les séquences des régions CDR des chaînes lourdes de l'anticorps produit par l'hybridome 18B6 Ainsi, la région CDR1 de chacune des chaînes légères de l'anticorps décrit dans la présente demande possède une séquence peptidique ayant au moins 70% d'identité avec la séquence de la région CDR1 des chaînes légères de l'anticorps produit par l'hybridome 18B6 , la région CDR2 de chacune des chaînes légères de l'anticorps décrit dans la présente demande possède une séquence peptidique ayant au moins 70% d'identité avec la séquence de la région CDR2 des chaînes légères de l'anticorps produit par l'hybridome 18B6, la région CDR3 de chacune des chaînes légères de l'anticorps décrit dans la présente demande possède une séquence peptidique ayant au moins 70% d'identité avec la séquence de la région CDR3 des chaînes légères de l'anticorps produit par l'hybridome 18B6, et la région CDR1 de chacune des chaînes lourdes de l'anticorps décrit dans la présente demande possède une séquence peptidique ayant au moins 70% d'identité avec la séquence de la région CDR1 des chaînes lourdes de l'anticorps produit par l'hybridome 18B6, la région CDR2 de chacune des chaînes lourdes de l'anticorps de la région CDR3 des chaînes légères de l'anticorps produit par l'hybridome 18B6 possède une séquence peptidique ayant au moins 70% d'identité avec la séquence de la région CDR2 des chaînes lourdes de l'anticorps produit par l'hybridome 18B6, et la région CDR3 de chacune des chaînes lourdes de l'anticorps décrit dans la présente demande possède une séquence peptidique ayant au moins 70% d'identité avec la séquence de la région CDR3 des chaînes lourdes de l'anticorps produit par l'hybridome 18B6 . De manière particulièrement avantageuse, l'identité avec chacune des séquences citées ci-dessus est d'au moins 80%, de manière préférée d'au moins 90%, 95%, 99% et de manière encore plus préférentielle de 100% d'identité.

Avantageusement, la région variable de chacune des chaînes légères de l'anticorps monoclonal anti-idiotypique décrit dans la présente demande est codée par une séquence d'acides nucléiques possédant au moins 70% d'identité avec la séquence d'acides nucléiques de la région variable des chaînes légères de l'anticorps produit par l'hybridome 18B6, et la région variable de chacune des chaînes lourdes de l'anticorps monoclonal anti-idiotypique est codée par une séquence d'acides nucléiques possédant au moins 70% d'identité avec la séquence d'acides nucléiques de la région variable des chaînes lourdes de l'anticorps produit par l'hybridome 18B6.

De manière particulièrement avantageuse, l'identité des séquences est d'au moins 80%, et de manière préférée d'au moins 95 à 99% d'identité. Le pourcentage d'identité est calculé en alignant 2 séquences à comparer et en comptant le nombre de positions possédant un nucléotide identique, ce nombre étant divisé par le nombre de nucléotides total de la séquence. La dégénérescence du code génétique peut être à l'origine du fait qu'un même acide aminé puisse être codé par plusieurs triplets de nucléotides différents. En tout état de cause, ces différences de séquences n'affectent en rien l'affinité de l'anticorps monoclonal pour sa cible, ni sa capacité à neutraliser l'activité inhibitrice des anticorps inhibiteurs cibles. Dans un aspect préféré de l'invention, la région variable de chacune des chaînes légères de l'anticorps monoclonal anti-idiotypique est codée par la séquence d'acide nucléique de la région variable des chaînes légères de l'anticorps produit par l'hybridome 18B6, et la région variable de chacune des chaînes lourdes de l'anticorps monoclonal anti-idiotypique est codée par la séquence d'acide nucléique de la région variable des chaînes lourdes de l'anticorps produit par l'hybridome 18B6. De manière avantageuse, la séquence peptidique de chacune des régions variables des chaînes légères de l'anticorps décrit dans la présente demande est une séquence possédant au moins 70% d'identité, et de manière avantageuse au moins 80% ou 90%, et de manière encore plus avantageuse au moins 99% d'identité avec la séquence peptidique de la région variable des chaînes légères de l'anticorps produit par l'hybridome 18B6.

De manière avantageuse, la séquence peptidique de chacune des régions variables des chaînes lourdes de l'anticorps décrit dans la présente demande est une séquence possédant au moins 70% d'identité, et de manière avantageuse au moins 80% ou 90%, et de manière encore plus avantageuse au moins 99% d'identité avec la séquence peptidique de la région variable des chaînes lourdes de l'anticorps produit par l'hybridome 18B6.

De manière préférentielle, la région variable de chacune des chaînes légères de l'anticorps monoclonal anti-idiotypique selon l'invention possède la séquence peptidique de la région variable des chaînes légères de l'anticorps produit par l'hybridome 18B6 et la région variable de chacune des chaînes lourdes de l'anticorps monoclonal anti-idiotypique selon l'invention possède la séquence peptidique de la région variable des chaînes lourdes de l'anticorps produit par l'hybridome 18B6. De manière préférentielle, l'anticorps inhibiteur cible de l'anticorps anti-idiotypique selon l'invention est l'anticorps RHD5, déposé à la Belgian Co-ordinated Collections of Microorganisms/Plasmid Collection (BCCM/LMBP), Laboratorium voor Moléculaire Biologie, University of Ghent, Technologiepark 297, B-9052 Zwijnaarede, Belgique en Août 2004, par la Collen Research Foundation, sous le numéro d'accession LMBP 6165CB. Cet anticorps, ainsi que ses séquences nucléotidiques et peptidiques sont décrits dans le document WO 2005/016455. L'anticorps RHD5 est un anticorps monoclonal humain IgG1 dirigé contre le domaine C1 du facteur VIII produit au départ de lymphocytes d'un patient présentant une hémophilie A, notamment une hémophilie A acquise, sévère avec un taux d'inhibiteurs élevé. Cet anticorps appartient à la sous classe IgG1) et est issu de la lignée germinale DP-10. L'épitope qu'il reconnaît sur le facteur VIII est le domaine C1 dans sa configuration native, mais pas le même domaine avec une mutation R2150H. L'anticorps RHD5 peut inhiber jusqu'à 98% l'activité du facteur VIII.

L'anticorps s'entend aussi de tout anticorps modifié répondant aux caractéristiques de l'invention, dans lequel un ou plusieurs acides aminés ont été substitué(s) ou délété(s). Une telle substitution ou délétion peut être localisée à n'importe quelle position dans la molécule. Dans le cas où plusieurs acides aminés ont été substitués ou délétés, toute combinaison de substitution ou de délétion peut être considérée. De telles altérations de la séquence des régions variables de l'anticorps selon l'invention peuvent être effectuées dans le but d'augmenter le nombre de résidus susceptibles d'entrer en contact entre l'anticorps anti-idiotypique selon l'invention et l'anticorps inhibiteur cible.

Dans un mode de réalisation de l'invention, l'anticorps anti-idiotypique est un anticorps murin.

Avantageusement, cet anticorps monoclonal anti-idiotypique murin est une IgG1kappa.

De manière préférée, l'anticorps monoclonal selon l'invention est un anticorps chimérique. On entend par « anticorps chimérique » un anticorps dont les régions variables des chaînes légères et des chaînes lourdes appartiennent à une espèce différente des régions constantes des chaînes légères et des chaînes lourdes. Ainsi, l'anticorps selon l'invention possède en outre des régions constantes de ses chaînes légères et lourdes appartenant à une espèce non-murine. A cet égard, toutes les familles et espèces de mammifères non-murins sont susceptibles d'être utilisées, et en particulier l'homme, le singe, les muridés (sauf la souris), les suidés, les bovidés, les équidés, les félidés, les canidés, par exemple, ainsi que les oiseaux.

Les anticorps chimériques selon l'invention peuvent être construits en utilisant les techniques standard de l'ADN recombinant, bien connues de l'homme du métier, et plus particulièrement en utilisant les techniques de construction des anticorps « chimériques » décrites par exemple dans Morrison et al., Proc. Natl. Acad. Sci. U.S.A., 81, pp. 6851-55 (1984), où la technologie de l'ADN recombinant est utilisée pour remplacer la région constante d'une chaîne lourde et/ou la région constante d'une chaîne légère d'un anticorps provenant d'un mammifère non-humain avec les régions correspondantes d'une immunoglobuline humaine.

Dans un aspect particulier de l'invention, l'anticorps selon l'invention est un anticorps hybride humain, c'est-à-dire un anticorps chimérique dont la partie constante est humaine. Ce mode de réalisation de l'invention permet de diminuer l'immunogénicité de l'anticorps chez l'homme et par là même d'améliorer son efficacité lors de son administration thérapeutique chez l'homme.

Avantageusement, l'anticorps selon l'invention est un anticorps humanisé. Un tel anticorps peut être obtenu par association d'une ou plusieurs région(s) CDR (Complementarity Determining Region) d'un anticorps monoclonal d'une espèce non-humaine avec des régions framework (régions très conservées des régions variables, nommées aussi "charpente") humaines, un tel procédé d'obtention étant bien enseigné dans l'état de la technique (Jones et al., Nature (1986) 321:522 ; Riechmann et al., Nature (1988) 332:323). Un tel anticorps humanisé, dirigé contre le domaine variable d'anticorps inhibiteurs reconnaissant le domaine C1 du FVIII, peut comporter des régions framework humaines et une ou plusieurs des régions CDR.

De manière avantageuse, l'anticorps monoclonal anti-idiotypique selon l'invention est l'anticorps 18B6 produit par l'hybridome 18B6 déposé sous le numéro d'enregistrement CNCM I-3559, le 24 janvier 2006 à la Collection Nationale de Cultures de Microorganismes (CNCM, 25 rue du Docteur Roux, 75724 Paris Cedex 15).

La méthode d'obtention de l'hybridome 18B6 est décrite dans la partie « Exemples » du présent document.

L'anticorps monoclonal anti-idiotypique selon l'invention s'entend aussi de tout anticorps comprenant des fragments de l'anticorps 18B6, et plus particulièrement tout anticorps comprenant la région variable de la chaîne légère et/ou la région variable de la chaîne lourde de l'anticorps 18B6, ou tout fragment de la région variable de la chaîne légère et/ou la région variable de la chaîne lourde de l'anticorps 18B6. Par « fragments » on entend désigner un fragment F'(ab')2 ou un fragment Fab' ou un fragment Fab ou une région CDR ou toute version modifiée de l'un quelconque de ces fragments ou région.

Dans un mode de réalisation particulier de l'invention, l'anticorps monoclonal anti-idiotypique selon l'invention est un fragment F(ab')2 ou un fragment Fab' ou un fragment Fab ou une région CDR ou toute version modifiée de l'un quelconque de ces fragments ou région. La digestion enzymatique des immunoglobulines par la papaïne génère 2 fragments identiques, qu'on appelle « fragment Fab » (Fragment Antigen Binding), et un fragment Fc (fraction cristallisable). Le fragment Fc est le support des fonctions effectrices des immunoglobulines.

Par digestion à la pepsine, un fragment F(ab')2 est généré, où les deux fragments Fab restent liés par deux ponts disulfure, et le fragment Fc est scindé en plusieurs peptides. Le fragment F(ab')2 est formé de deux fragments Fab' (un fragment Fab' consistant en un Fab et une région charnière), liés par des ponts disulfure intercaténaires pour former un F(ab')2.

De tels fragments, qui contiennent le site de liaison de l'anticorps, peuvent avoir perdu un certain nombre de propriétés de l'anticorps entier duquel ils sont issus, comme la capacité d'activer le complément ou de lier les récepteurs Fcgamma. Toutefois, ces fragments n'ont pas perdu la capacité de l'anticorps entier de neutraliser l'anticorps inhibiteur. Ainsi, l'invention s'entend aussi des fragments F(ab')2, Fab', Fab, de la région CDR ou toute version modifiée de l'un quelconque de ces fragments ou région, de l'anticorps 18B6. En particulier, ces fragments ont conservé la capacité de l'anticorps entier de neutraliser les anticorps RHD5.

Un autre objet de l'invention est une lignée cellulaire stable produisant un anticorps tel que décrit précédemment. La lignée cellulaire stable selon l'invention peut être d'origine humaine ou animale. La lignée cellulaire stable selon l'invention peut provenir de cellules humaines immortalisées. Dans un autre mode de réalisation de l'invention, cette lignée peut provenir de cellules d'origine animale, par exemple de souris, immortalisées. Un exemple préféré d'une lignée issue de ce mode de réalisation de l'invention est la lignée 18B6, déposée à la CNCM sous le numéro I-3559. Dans un autre mode de réalisation, la lignée cellulaire stable selon l'invention est une lignée qui a intégré une construction génétique permettant l'expression de l'anticorps selon l'invention à l'endroit désiré du génome. L'étape qui consiste à obtenir une telle cellule est une transfection stable. Cette étape peut être appliquée sur n'importe quel type de cellules pourvu qu'elles puissent être maintenues en culture in vitro. La transfection stable nécessite l'intégration de la construction génétique, qui peut être effectuée par recombinaison homologue ou peut se faire de manière aléatoire. C'est la présence d'une cassette de sélection positive dans la construction génétique comportant le gène d'intérêt qui confère à la cellule la résistance à un antibiotique par exemple qui atteste de l'insertion du transgène dans le génome cellulaire. A l'issue d'une étape de sous-clonage, on obtient une lignée cellulaire productrice à long terme de l'anticorps de l'invention, par exemple 18B6, pouvant être maintenue en culture in vitro.

La lignée cellulaire stable exprimant un anticorps selon l'invention peut être choisie parmi le groupe consistant en une lignée cellulaire humaine, une lignée de rongeur, par exemple une lignée murine, SP2/0, YB2/0, IR983F, un myélome humain comme Namalwa ou toute autre cellule d'origine humaine comme PERC6, les lignées CHO, notamment CHO-K-1, CHO-Lec10, CHO-Lec1, CHO-Lec13, CHO Pro-5, CHO dhfr- (CHO DX B11, CHO DG44), ou d'autres lignées choisies parmi wil-2, Jurkat, Vero, Molt-4, COS-7, 293-HEK, BHK, K6H6, NS0 SP2/0-Ag 14 et P3X63Ag8.653.

Un autre objet particulier de l'invention est l'hybridome 18B6 déposé sous le numéro d'enregistrement CNCM I-3559 à la Collection Nationale de Cultures de Microorganismes (CNCM).

L'anticorps produit par l'hybridome 18B6 est l'anticorps 18B6, et une méthode d'obtention de l'hybridome 18B6 est décrite dans la partie « Exemples » du présent document. Un autre objet est un fragment d'ADN codant pour la région variable de la chaîne lourde de l'anticorps selon l'invention tel que décrit précédemment. Ce fragment d'ADN peut être inséré dans un vecteur permettant l'expression d'un polypeptide, préférentiellement d'un anticorps, dont la région variable de la chaîne lourde est codée par la séquence d'acide nucléique encodant la région variable des chaînes lourdes de l'anticorps produit par l'hybridome 18B6, dont la séquence peptidique déduite est la séquence peptidique de la région variable des chaînes lourdes de l'anticorps produit par l'hybridome 18B6, afin de l'introduire et de le maintenir dans une cellule hôte.

Un autre objet de l'invention est une composition pharmaceutique comprenant un anticorps selon l'invention et au moins un excipient et/ou au moins un véhicule pharmaceutiquement acceptable. De manière préférentielle, l'anticorps monoclonal anti-idiotypique contenu dans la composition pharmaceutique selon l'invention est l'anticorps 18B6, un fragment ou région dérivée de 18B6, ou encore un anticorps chimérique ou humanisé comportant les régions variables ou les CDR de 18B6 et tel que défini dans les revendications. La composition pharmaceutique selon l'invention peut être formulée dans tout excipient qu'un patient à traiter peut tolérer. Des exemples de tels excipients incluent l'eau, les solutions salines, la solution de Ringer, les solutions de dextrose, et toute autre solution aqueuse physiologique appropriée. L'excipient peut aussi contenir de faibles quantités d'additifs tels que des substances qui augmentent l'isotonicité et la stabilité de la composition. De tels excipients incluent le tampon phosphate, le tampon bicarbonate, et le tampon Tris. De tels excipients sont bien connus de l'homme du métier. Des formulations standard peuvent se présenter sous forme de liquides injectables ou de formulations solides qui peuvent être re-suspendues dans un liquide approprié avant administration. Les véhicules pouvant être utilisés pour la préparation de la composition pharmaceutique selon l'invention ont avantageusement pour fonction d'augmenter la demi-vie de la composition thérapeutique dans l'animal ou le patient, ou pour permettre le relargage contrôlé du principe actif. De tels véhicules peuvent être des polymères organiques et synthétiques et d'autres composés chimiques qui peuvent disséminer les médicaments à une cadence normale ou les disséminer seulement dans certains environnements, ainsi que des liposomes, cette liste n'étant pas limitative. Avantageusement, la composition pharmaceutique comprend en outre au moins un anticorps anti-idiotypique dirigé contre un anticorps inhibiteur se liant à un domaine autre que le domaine C1 du facteur VIII. Cet autre anticorps peut être un anticorps anti-idiotypique dirigé contre un anticorps inhibiteur se liant au domaine A1, ou A3, ou B, A2 ou C2 du facteur VIII. En effet, un patient atteint d'hémophilie A ayant développé des anticorps inhibiteurs présente le plus souvent plusieurs types d'anticorps inhibiteurs. De plus, les quantités et la nature des différents types d'anticorps inhibiteurs ne sont pas fixes mais peuvent changer au cours de la vie du patient. Les différents anticorps inhibiteurs d'un même patient étant ainsi dirigés contre des domaines différents du facteur VIII, il est particulièrement avantageux de traiter le patient non pas avec un, mais avec plusieurs types d'anticorps anti-idiotypiques, dirigés contre des anticorps inhibiteurs différents.

La composition pharmaceutique comporte un anticorps monoclonal anti-idiotypique dirigé contre un anticorps inhibiteur se liant au domaine C2 du facteur VIII et/ou un anticorps inhibiteur se liant au domaine A2 du facteur VIII et l'anticorps monoclonal selon l'invention. En effet, les domaines A2 et C2 représentent les cibles principales de la réaction immunitaire anti-facteur VIII. Ainsi, une composition pharmaceutique comportant un mélange d'anticorps anti-idiotypiques dirigés contre des anticorps inhibiteurs se liant au domaine C1 du facteur VIII et d'anticorps anti-idiotypiques dirigés contre des anticorps inhibiteurs se liant au domaine C2 permet la neutralisation d'au moins 70%, et avantageusement au moins 80% ou 90% de la totalité des anticorps inhibiteurs présents chez un patient. Dans un mode de réalisation préféré de l'invention, la composition pharmaceutique selon l'invention comporte aussi l'anticorps 14C12 (déposé sous le numéro LMBP 5878CB à la Belgian Coordinated Collections of Microorganisms) et/ou l'anticorps 30D1 (déposé à la CNCM sous le numéro I-3450). Dans un autre mode de réalisation préféré de l'invention, la composition pharmaceutique comporte aussi l'anticorps 14C12 chimérique déposé à la CNCM sous le numéro I-3510 et/ou un anticorps chimérique ou humanisé dérivé de l'anticorps 30D1, c'est-à-dire un anticorps comportant les régions variables de l'anticorps 30D1.

Un autre objet de l'invention est l'utilisation de l'anticorps selon l'invention pour son utilisation comme médicament.

Un autre objet, de l'invention est l'utilisation de l'anticorps selon l'invention pour la fabrication d'un médicament. Avantageusement, un tel médicament est utilisé pour réduire et/ou prévenir et/ou traiter les saignements chez un patient atteint d'hémophilie comportant des anticorps inhibiteurs dirigés contre le domaine C1 du facteur VIII.

Un autre objet de l'invention est l'utilisation de l'anticorps selon l'invention pour la fabrication d'un médicament destiné au traitement de l'hémophilie de type A.

Avantageusement, l'hémophilie de type A ainsi traitée est une hémophilie avec inhibiteurs. Ce type d'hémophilie traitée par l'anticorps selon l'invention peut être innée ou acquise. L'anticorps selon l'invention, en neutralisant les anticorps inhibiteurs, rend son efficacité au traitement par injection de facteur VIII au patient, l'activité du facteur VIII n'étant plus inhibée par les anticorps inhibiteurs.

Un autre objet de l'invention est l'utilisation de l'anticorps selon l'invention pour neutraliser *in vitro* ou in vivo l'activité inhibitrice d'un anticorps inhibiteur dirigé contre le domaine C1 du facteur VIII. Ce procédé peut être mis en oeuvre pour dépléter le sang d'un patient de ses anticorps inhibiteurs dirigés contre le domaine C1 du facteur VIII, le sang étant ensuite réinjecté audit patient.

Un autre objet de l'invention se rapporte à un médicament comprenant un anticorps selon l'invention, préférentiellement l'anticorps 18B6.

Un autre objet de l'invention est l'utilisation de l'anticorps pour adsorber les anticorps inhibiteurs, à titre d'exemple afin de purifier des anticorps inhibiteurs du facteur VIII.

Enfin, un autre objet de l'invention est l'utilisation de l'anticorps selon l'invention pour la détection et/ou la purification d'anticorps inhibiteurs du facteur VIII. Les procédés de mise en oeuvre de telles méthodes de détection et de purification sont bien connus de l'homme du métier. On peut citer comme exemple l'utilisation à cet effet d'une colonne d'immuno-purification contenant des billes à la surface desquelles est greffé l'anticorps selon l'invention. Seules les molécules reconnues par l'anticorps vont se fixer sur les billes. Les autres vont traverser la colonne. Pour récupérer la molécule, il suffit d'augmenter la force ionique du solvant.

D'autres aspects et avantages de l'invention seront décrits dans les exemples qui suivent, qui doivent être considérés comme illustratifs et ne limitent pas l'étendue de l'invention.

## Description des Figures

> **Figure 1** : augmentation (valeur moyenne) pour les 4 souris de la fixation des anticorps anti-idiotypiques sur le RHD5.
> **Figure 2** : fixation directe de l'anticorps anti-idiotypique 18B6 à l'anticorps RHD5 insolubilisé.

**Figure 3** : inhibition de la fixation de l'anticorps RHD5 au FVIII recombinant (recFVIII) insolubilisé **Figure 4** : neutralisation du RHD5 par le 18B6

## Exemples

### Exemple 1 : Production d'un anticorps monoclonal humain dirigé contre le domaine C1 du facteur VIII (« anticorps anti-C1 »)

La lignée cellulaire lymphoblastoïde humaine RHD5 décrite ci-dessous a été obtenue par immortalisation des lymphocytes B d'un patient souffrant d'hémophilie A acquise ayant développé une réponse immune contre le facteur VIII, conformément à la procédure décrite dans le document Jacquemin et al. (1998), Blood 92, 496-506 et dans le document WO 2005/016455.

La lignée cellulaire produisant l'anticorps monoclonal anti-C1 RHD5 a été déposée à la Belgian Co-ordinated Collections of Microorganisms/Plasmid Collection (BCCM/LMBP), Laboratorium voor Moleculaire Biologie, University of Ghent, Technologiepark 297, B-9052 Zwijnaarede, Belgium en Août 2004, par la Collen Research Foundation, sous le numéro d'accession LMBP 61-65CB.

La séquence nucléotidique de la région variable de la chaîne lourde de l'anticorps RHD5 est la séquence SEQ ID NO : 5, et la séquence nucléotidique de la région variable de la chaîne légère de l'anticorps RHD5 est la séquence SEQ ID NO : 6. La séquence peptidique correspondant à la séquence SEQ ID NO : 5 est la séquence SEQ ID NO : 7 et la séquence peptidique correspondant à la séquence SEQ ID NO : 6 est la séquence SEQ ID NO : 8.

Alternativement, des anticorps possédant les caractéristiques requises peuvent être produits par immunisation d'animaux. Dans ce cas, le facteur VIII humain est injecté à des souris avec un adjuvant. Les anticorps monoclonaux anti-humain sont ensuite obtenus par fusion de lymphocytes de rate avec une lignée cellulaire de myélome de souris. Les surnageants cellulaires produisant les anticorps anti-facteur VIII sont identifiés et clonés par dilution limite. Une description générale de telles méthodes peut être trouvée dans « Current Protocols in Immunology, Chapter 2, John Wiley & Sons, Inc, 1994 ». D'autres sélections d'inhibiteurs possédant les caractéristiques désirées sont décrites ci-dessous.

### Exemple 2 : Production de l'anticorps anti-idiotypique 18B6

#### I. Immunisation des souris

Quatre souris Balb/c femelles âgées de 6 semaines ont été injectées en sous-cutané (SC) dans les coussinets plantaires à trois reprises avec 10 $\mu$g de l'anticorps humain anti-domaine C1 du FVIII RHD5 suspendu dans un adjuvant complet de Freùnd (ACF) (1$^{ère}$ immunisation) puis incomplet (AIF).

Une première saignée (saignée 0) à été effectuée avant immunisation (saignée à Jour 0 (J0)), puis les injections et saignées se sont succédées comme suit :

J1: Injection N°1 (10 $\mu$g d'anticorps RHD5 en présence d'adjuvant complet de Freund)
J15: Saignée N°1
J16: Injection N°2 (10 $\mu$g d'anticorps RHD5 en présence d'adjuvant incomplet de Freund)
J28: Saignée N°2
J29: Injection N°3 (10 $\mu$g d'anticorps RHD52 en présence d'adjuvant incomplet de Freund)
J44: Saignée N°3

#### II. Evaluation de la réponse immune des souris

De manière à évaluer la présence d'anticorps anti-RHD5 dans les différentes saignées, un test ELISA en fixation directe est pratiqué. Dans ce but, on a insolubilisé soit l'anticorps RHD5, soit une IgG1 contrôle à 3 $\mu$g/ml, 50 $\mu$l/puits, dans du Tampon Glycine, sur la nuit (over night) à 4°C (Tampon Glycine = Glycine 0,1M, NaCl 0,17M, pH 9.2). Trois lavages sont effectués avec du PBS/Tween (PBS = NaCl 140,0 mM, KCl 2,6 mM, KH$_2$PO$_4$ 1,4 mM, Na$_2$HPO$_4$. 2H$_2$O 8,1 nM, pH 7.4). Le système est laissé à saturation pendant 30 minutes à température ambiante (RT) avec 100 $\mu$l/puits de Magic Buffer (Magic Buffer = Tris 50mM, Nacl 0,17M, BSA 1%, pH 7.2). Les saignées sont ensuite diluées à 1/10, 1/100, 1/1000 et 1/10000 dans le Magic Buffer et incubées pendant 2 heures à température ambiante (50$\mu$l/ puits). Puis 3 lavages sont effectués dans du PBS/Tween. Le système est ensuite incubé avec une solution d'anticorps polyclonaux de chèvre anti-IgG de souris marqués à la HRP (horseradish peroxidase) (Bio-Rad) à 1 $\mu$g/ml pendant 2 heures à température ambiante (50$\mu$l/puits) (dilution dans le Magic Buffer). Le système est ensuite lavé 3 fois avec du PBS/Tween, puis on effectue une révélation avec un chromogène (Ortho-phényl diamine) et une lecture de l'intensité de la coloration obtenue par un lecteur avec les filtres correspondant aux longueurs d'ondes 490/650 nm (lecteur Emax Molecular Devices, Sunnyvale, CA).

Résultat des densités optiques obtenues sur l'IgG1 contrôle:

Tableau 1

| Dilution 1000X | Souris 1 | Souris 2 | Souris 3 | Souris 4 |
|---|---|---|---|---|
| Saignée 0 | 0,031 | 0,019 | 0,018 | 0,018 |
| Saignée 1 | 0,019 | 0,020 | 0,025 | 0,028 |
| Saignée 2 | 0,026 | 0,023 | 0,169 | 0,045 |
| Saignée 3 | 0,027 | 0,063 | 0,150 | 0,024 |

Résultat des densités optiques obtenues sur le RHD5 :

Tableau 2

| Dilution 1000X | Souris 1 | Souris 2 | Souris 3 | Souris 4 |
|---|---|---|---|---|
| Saignée 0 | 0,047 | 0,020 | 0,012 | 0,018 |
| Saignée 1 | 0,446 | 0,188 | 0,142 | 0,157 |
| Saignée 2 | 0,632 | 0,685 | 0,648 | 0,911 |
| Saignée 3 | 0,570 | 0,708 | 0,778 | 0,852 |

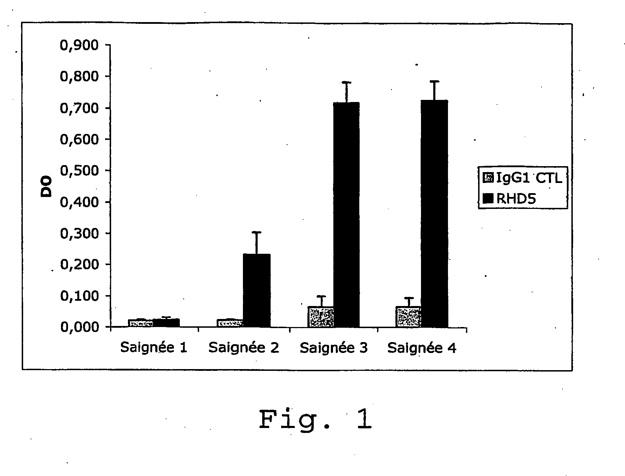

Les résultats obtenus sont reportés dans la figure 1. Conclusion : Chaque souris a répondu correctement et de façon similaire à l'injection du fragment Fab RHD5. De façon arbitraire, la souris n°4 a été sélectionnée pour faire la fusion.

III. Fusion et criblage

Les lymphocytes de la rate de la souris n°4 ont été fusionnés avec les cellules d'un myélome SP2/0. La fusion a été réalisée de manière classique pour l'homme du métier (J.G. Gilles et al., Blood (2004) 103 : 2617-23 ; P. Cornelis, « Les anticorps monoclonaux », Revue IRE, vol. 7, N°4, 1983).

Les cellules ont été expandues successivement en milieu DMEM (Dulbecco's Modified Eagle Medium) contenant de l'hypoxanthine et de la thymidine selon le principe des dilutions limites et les clones testés positifs détectés par un test en fixation directe ELISA tel que décrit précédemment au point II.

La spécificité de la fixation a été confirmée par l'insolubilisation d'un anticorps IgG1 humain de spécificité non relevante produit par notre laboratoire. Afin de déterminer si les hybridomes sont stables, nous avons répété le criblage épitopique (tests 1 à 3) au cours de l'expansion des clones, dans différents volumes de milieu allant de 200 μl à 5 ml.

Test 1 = mesure dans puits de 200 μl
Test 2 = mesure dans puits de 1 ml
Test 3 = mesure dans bouteille de 5 ml

Les résultats obtenus lors des différents criblages épitopiques figurent dans le tableau suivant :

Tableau 3

| | Criblage 1 | Criblage 2 | Criblage 3 | Criblage : 3 /IgG1 | Inhibition: Elisa | Neutralisation : test fonctionnel |
|---|---|---|---|---|---|---|
| 1A1 | + | - | - | - | | |
| **1F3** | + | + | + | - | + (92.5%) | + (100%) |
| 2A1 | + | - | - | - | | |
| 2C9 | + | + | + | + | | |
| 3G9 | + | + | + | + | | |
| 4B7 | + | + | - | - | | |

(suite)

| | Criblage 1 | Criblage 2 | Criblage 3 | Criblage : 3 /IgG1 | Inhibition: Elisa | Neutralisation : test fonctionnel |
|---|---|---|---|---|---|---|
| 4B10 | + | + | + | + | | |
| 4D5 | + | + | + | + | | |
| **5B11** | + | + | + | - | + (93.6%) | + (100%) |
| 5E1 | + | - | +/- | + | | |
| 5G3 | + | + | + | + | | |
| 5H7 | + | - | - | + | | |
| 5H8 | + | + | +/- | + | | |
| 6A9 | + | + | + | + | | |
| 6E1 | + | + | +/-- | + | | |
| 6H7 | + | + | + | + | | |
| 6H8 | + | + | + - | + | | |
| 9D2 | + | - | - | + | | |
| **10D2** | + | + | + | - | + (93.6%) | + (100%) |
| **10G8** | + | + | + | - | - | |
| **11C5** | + | + | + | - | - | |
| 11D7 | + | - | - | - | | |
| 11G3 | + | + | + | + | | |
| **12D7** | + | + | + | - | - | |
| 12G3 | + | - | - | - | | |
| **12H12** | + | + | + | - | - | |
| **13A1** | + | + | + | - | + (90.9%) | + (95.6%) |
| **13C3** | + | + | + | - | + (93.6%) | + (100%) |
| 13D7 | + | + | + | - | | |
| 13H5 | + | + | +/- | + | | |
| 14H1 | + | + | - | - | | |
| 14D11 | + | + | +/-- | + | | |
| **14F11** | + | + | + | - | + (83.6%) | + (100%) |
| 14H2 | + | + | + | + | | |
| 14H5 | + | + | + | + | | |
| 15B4 | + | + | - | - | | |
| 15F6 | + | - | - | + | | |
| **16B4** | + | + | + | - | + (94%) | + (90.9%). |
| **16F6** | + | + | + | - | - | |
| 17A5 | + | + | + | + | | |
| **17C4** | + | + | + | - | + (92.4%) | + (100%) |
| **18A5** | + | + | + | - | - | |
| **18A9** | + | + | + | - | - | |

(suite)

|  | Criblage 1 | Criblage 2 | Criblage 3 | Criblage : 3 /IgG1 | Inhibition: Elisa | Neutralisation : test fonctionnel |
|---|---|---|---|---|---|---|
| **18B6** | + | + | + | - | + (93.1%) | + (100%) |
| 18C4 | + | + | + | + |  |  |
| 19C4 | + | + | + | + |  |  |
| 19G3 | + | + | - | +/- |  |  |
| 20A7 | + | + | + | + |  |  |
| 20C4 | + | - | - | - |  |  |
| 20G3 | + | - | - | - |  |  |
| **21D8** | + | + | + | - | + (93.8%) | - |
| 22H7 | + | - | + | + |  |  |
| **23A7** | + | + | + | - | - |  |
| 23E3 | + | + | - | - |  |  |
| **23G2** | + | - | +/- | - | - |  |
| **24D5** | + | + | + | - | +/-(56.3%) | +/- (76.9%) |
| 24D12 | + | + | + | + |  |  |
| 24E3 | + | - | - | - |  |  |
| 28C10 | + | - | - | - |  |  |

IV. Test d'inhibition avec les surnageants de culture

Comme le montre le tableau 3, un test d'inhibition a été réalisé avec les surnageants de culture. Ce test est réalisé afin de sélectionner parmi les clones, les anticorps anti-idiotypiques qui reconnaissent précisément un déterminant épitopique localisé au niveau du paratope de l'Ac RHD5. Nous avons ainsi testé les anticorps anti-idiotypiques dans un test ELISA d'inhibition de fixation du RHD5 au FVIII insolubilisé.

Le facteur VIII recombinant (recFVIII) (Baxter) à 2 $\mu$g/ml dans un tampon glycine, 50 $\mu$l/puits, a été insolubilisé puis laissé pendant 2 heures à température ambiante. L'anticorps RHD5 (ou une IgG1 non relevante) à 0,6 $\mu$g/ml final a été pré-incubé pendant 2 heures avec les surnageants de culture à la dilution 1/1, 1/2 et 1/4 dans le Magic Buffer. Un lavage des puits à 3 reprises est effectué avec un tampon PBS/Tween, puis une saturation avec 100 $\mu$l/puits de Magic Buffer (30 min à température ambiante) est effectuée. On incube ensuite avec 50 $\mu$l de RHD5 (ou d'IgG1 non relevante) le surnageant de culture (2 heures à température ambiante, Magic Buffer), puis trois lavages sont effectués. Les anticorps RHD5 fixés au recFVIII insolubilisé sont détectés par ajout de 50 $\mu$l/puits d'une solution à 1 $\mu$g/ml dans le Magic Buffer d'anticorps polyclonaux de souris anti-IgG humaines marqués-HRP (Southern Biotechnology). Trois lavages successifs sont effectués avec du PBS/Tween, puis on effectue la révélation avec un chromogène (OPD ortho-phényl diamine) et une lecture de l'intensité de la coloration obtenue par un lecteur avec les filtres correspondant aux longueurs 490/650 nm (lecteur Emax Molecular Devices, Sunnyvale, CA).

Conclusions :

Comme le montre le tableau 3, 11 clones sont capables d'inhiber spécifiquement la fixation de l'anticorps RHD5 au recFVIII insolubilisé. (N.B. : Une valeur négative traduit soit la possibilité d'une fixation sur une région externe du paratope soit est le reflet d'une concentration insuffisante de l'Ac dans le surnageant de culture. Cependant, vu le nombre de positifs, les puits négatifs ont été éliminés de la suite des essais).

V. Test fonctionnel avec les surnageants de culture mesure de la neutralisation de l'activité inhibitrice (anti-facteur VIII) de l'anticorps RHD5

L'anticorps RHD5 est incubé à la concentration de 1 $\mu$g/ml avec les surnageants des différents clones sélectionnés lors du test d'inhibition (dilutions 3 fois, 6 fois, 12 fois et 24 fois) dans du Magic Buffer à 37°C. Après 30 min, le FVIII Kogenate (Bayer) à 0,5 U/ml final est ajouté puis une incubation complémentaire de 30 min à 37°C est effectuée. Les échantillons sont dilués 30X dans le Magic Buffer, puis les réactifs du test Chromogénique DADE (facteur VIII chromogénique, Dade Behring Gmbh, Marburg, Germany) sont ajoutés selon le mode d'emploi du fabricant.

Comme le montre le tableau 3, 10 clones sont capables de neutraliser l'activité inhibitrice de l'Ac RHD5. En fonction des résultats et des courbes de neutralisation, l'anticorps 18B6 est sélectionné pour les expériences à suivre.

VI. Production de façon extensive du clone 18B6 nanti-RHD5 sélectionné

L'anticorps anti-idiotypique 18B6 a été produit en milieu de culture DMEM. Cette production a été suivie d'une purification sur colonne d'affinité Protéine G (ce qui permet de purifier puis de concentrer les anticorps et ainsi de s'assurer plus avant de la spécificité de l'anticorps anti-idiotypique obtenu).

Purification : 18B6 : production de 8 ml à 8,48 mg/ml

VII. Evaluation de la spécificité

Les différentes préparations sont évaluées en ELISA suivant le même protocole que celui décrit au point II.et IV.

1. Test ELISA : fixation directe de l'anticorps anti-idiotypique 18B6 à l'anticorps RHD5 insolubilisé

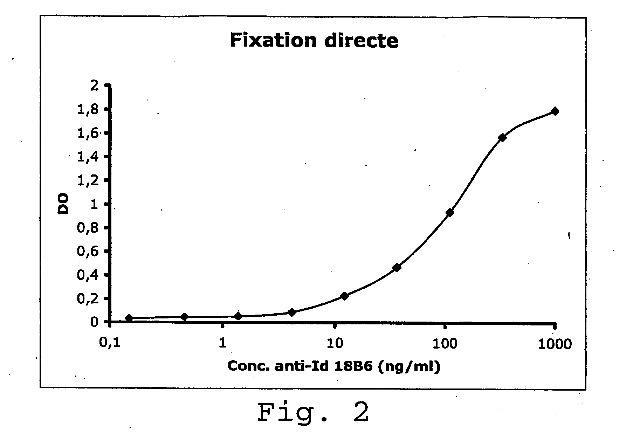

La fixation directe de l'anticorps anti-idiotypique 18B6 à l'anticorps RHD5 insolubilisé est montrée à la figure 2. La courbe montre que la fixation de l'anticorps 18B6 sur le RHD5 est dose dépendante.

2. Test ELISA : inhibition de la fixation de l'anticorps RHD5 au FVIII recombinant insolubilisé

L'inhibition de la fixation de l'anticorps RHD5 au FVIII recombinant insolubilisé au été mesurée, selon le protocole décrit au point IV. La concentration de RHD5 utilisée est égale à 2 $\mu$g/ml.

Tableau 4

| Conc. 18B6 $\mu$g/ml | Inhibition de la fixation (%) |
|---|---|
| 50 | 96,1 |
| 25 | 97,2 |
| 12,5 | 96,9 |
| 6,25 | 96,7 |
| 3,12 | 94,1 |
| 1,56 | 90,3 |
| 0,78 | 50,3 |
| 0,39 | 9,2 |
| 0,195 | 0 |
| 0,098 | 0 |

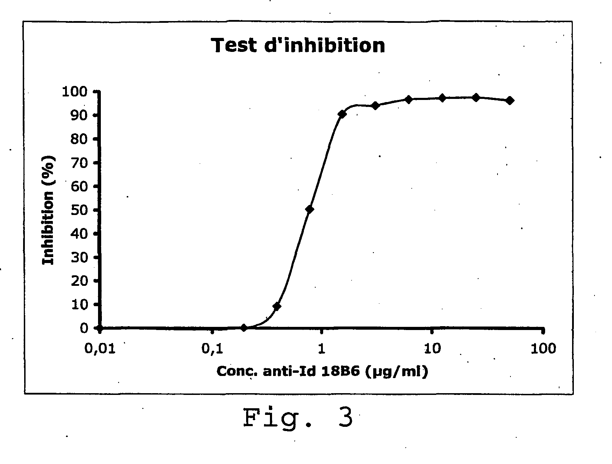

Les résultats sont montrés à la figure 3. 50% de l'inhibition de la fixation du RHD5 au FVIII est obtenue à un rapport molaire RHD5/18B6 de 2,5 alors qu'un rapport équimolaire inhibe 92% de cette fixation.

3. Test fonctionnel : mesure de la neutralisation de l'activité inhibitrice (anti-FVIII) de l'anticorps RHD5 Le protocole est identique à celui décrit au point V avec une concentration finale de RHD5 de 0,4 $\mu$g/ml et une courbe de l'anti-idiotypique purifié allant de 4 à 0,002 $\mu$g/ml final.

Les résultats sont donnés dans le tableau 5 suivant :

Tableau 5

| Conc. anti-Id ($\mu$g/ml) | Neutralisation (%) |
|---|---|
| 4 | 89,5 |
| 1,33 | 81,2 |
| 0,44 | 54,3 |
| 0,148 | 27,4 |
| 0,049 | 11 |

(suite)

| Conc. anti-Id ($\mu$g/ml) | Neutralisation (%) |
|---|---|
| 0,0165 | 8,8 |
| 0,0055 | 6,7 |
| 0,00183 | 8,9 |
| 0,0006 | 6,7 |
| 0,0002 | 0 |

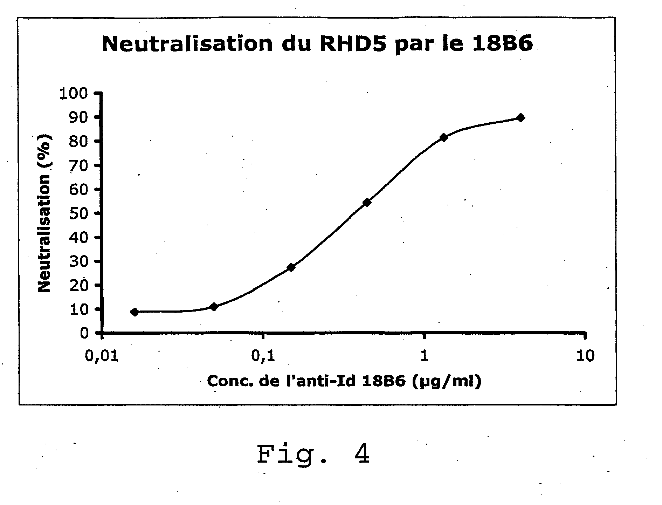

Les résultats sont montrés à la figure 4. 50% de neutralisation de l'activité inhibitrice du RHD5 est obtenue à un rapport RHD5/18B6 équimolaire.

4. Mesure de la cinétique de fixation de l'anticorps anti-idiotypique 18B6 par la méthode « Surface plasmon résonance Biacor »

La cinétique de fixation de l'anticorps anti-idiotypique 18B6 sur l'anticorps inhibiteur RHD5 a été évaluée par la méthode « Surface plasmon résonance Biacore » en utilisant le Pharmacia Biosensor BIAcore (Pharmacia Biosensor AB, Uppsala, Suède). Les anticorps RHD5 ont été immobilisés sur une surface activée d'une sonde CM5. Les anticorps anti-idiotypiques 18B6 ont été infusés avec plusieurs concentrations de RHD5 immobilisés sur la surface de la sonde. Les constantes d'association et de dissociation ont été déterminées :

$$K_a \ (M\text{--}1S\text{--}1) = 4,26 \ \times \ 10^3$$

$$K_d \ (S\text{--}1) = 1,45 \ \times \ 10^{-5}$$

$$K_D :M. \ 3,4 \ \times \ 10^{-9}$$

5. Caractérisation de la sous-classe de l'anticorps anti-idiotypique 18B6

Afin de déterminer la sous-classe de l'anticorps 18B6, le système IsoStrip de Roche a été utilisé (languette colorimétrique). L'anticorps 18B6 est une IgG1, Kappa.

FVIII. <u>Séquence de l'anticorps 18B6</u>

Pour le séquençage, l'ARNm des hybridomes produisant l'anticorps anti-idiotypique 18B6 a été isolé en utilisant le Quick Prep Micro mRNA Purification Kit (Amersham Pharmacia Biotech, Uppsala, Suède). L'ADNc a été synthétisé au moyen du First-strand cDNA Synthesis Kit (Amersham Pharmacia Biotech). L'ADNc codant pour la chaîne lourde (VH) et pour la chaîne légère (VL) a été amplifié par PCR (Polymerase Chain Reaction) au moyen d'amorces spécifiques correspondant aux différentes familles de gènes potentiellement rencontrées chez la souris. Les produits de la PCR ont été isolés d'un gel d'agarose 1,5% au moyen du QIA quick Gel Extraction Kit (Qiagen, Hilden, Germany) et clonés au moyen du pGEM-T Easy Vector system (Promega, Madison, WI). L'ADN plasmidique des colonies positives a été isolé au moyen de High Pure Plasmid Isolation Kit (Roche Diagnostics, Mannheim, Germany) et séquencé dans les deux sens avec la Seqenase (US Biochemical, Cleveland, OH).

IX. <u>Propriétés particulières liées à l'anticorps 18B6</u>

L'anticorps 18B6 inhibe complètement la fixation de l'anticorps RHD5 à son antigène, le Facteur VIII. L'anticorps RHD5 porte une idiotype complémentaire à celui du 18B6.

La fixation d'un anticorps à l'antigène concerne une interface de reconnaissance mutuelle de 6 à 12 angströms$^2$, correspondant à un grand nombre d'acides aminés s'associant l'un avec l'autre à l'aide de liens hydrogènes, d'attraction hydrophobe ou polaire et de ponts de VanderWals.

Sur un plan fonctionnel, lorsque qu'un anticorps inhibe complètement la fixation d'un anticorps à l'antigène, cela implique que l'anticorps inhibant porte une "image interne" de l'antigène, à savoir une structure tridimensionnelle mimant la structure 3-D de l'antigène.

Bien que la structure primaire (séquence en acides aminés) de 18B6 offre une faible homologie avec celle du domaine C1 du facteur VIII, l'alignement des structurels secondaires de l'anticorps 18B6 avec celle du domaine C1 du FVIII, cible antigénique de l'anticorps RHD5, ainsi que la modélisation tridimensionnelle du 18B6 indiquent, par superposition avec la structure 3-D du domaine C1, que la partie variable de la chaine légère (VL) du 18B6 représente une image interne du domaine C1.

Cette observation confère à l'anticorps 18B6 une propriété particulière, nouvelle et non prévisible. En d'autres termes, toute tentative de générer des anticorps semblables au 18B6 par immunisation avec un anticorps tel que RHD5 ne résulte pas de facto en l'obtention d'anticorps identiques à 18B6.

## Revendications

1. Anticorps monoclonal anti-idiotypique dirigé contre un anticorps humain inhibiteur du facteur VIII, ledit anticorps inhibiteur étant dirigé contre le domaine C1 du facteur VIII, dans lequel la région variable de chacune de ses chaînes légères est codée par la séquence d'acide nucléique codant la région variable de la chaîne légère de l'anticorps produit par l'hybridome 18B6, déposé sous le numéro d'enregistrement CNCM I-3559 à la Collection Nationale de Cultures de Microorganismes (CNCM), et la région variable de chacune de ses chaînes lourdes est codée par la séquence d'acide nucléique codant la région variable de la chaîne lourde de l'anticorps produit par l'hybridome 18B6, déposé sous le numéro d'enregistrement CNCM I-3559 à la Collection Nationale de Cultures de Microorganismes (CNCM).

2. Anticorps monoclonal anti-idiotypique selon la revendication 1, dans lequel la séquence peptidique de la région variable de chacune de ses chaînes légères est la séquence de la région variable de la chaîne légère de l'anticorps produit par l'hybridome 18B6, déposé sous le numéro d'enregistrement CNCM I-3559 à la Collection Nationale de Cultures de Microorganismes (CNCM), et la séquence peptidique de la région variable de chacune de ses chaînes lourdes est la séquence de la région variable de la chaîne lourde de l'anticorps produit par l'hybridome 18B6, déposé sous le numéro d'enregistrement CNCM I-3559 à la Collection Nationale de Cultures de Microorganismes (CNCM).

3. Anticorps monoclonal anti-idiotypique selon l'une quelconque des revendications 1 à 2, **caractérisé en ce que** l'anticorps inhibiteur est l'anticorps RHD5 (déposé à la collection BCCM/LMBP sous le numéro LMBP 6165CB).

4. Anticorps monoclonal anti-idiotypique selon l'une quelconque des revendications 1 à 2, **caractérisé en ce qu'**il est un anticorps murin ou un anticorps chimérique, notamment un anticorps hybride humain ou un anticorps humanisé.

5. Anticorps monoclonal anti-idiotypique selon la revendication 4, **caractérisé en ce qu'**il est une IgG1kappa.

6. Anticorps monoclonal anti-idiotypique selon l'une quelconque des revendications 1 à 5, **caractérisé en ce qu'**il s'agit d'un fragment F(ab)2, d'un fragment Fab', d'un fragment Fab.

7. Anticorps monoclonal anti-idiotypique selon l'une quelconque des revendications 1 à 6, **caractérisé en ce qu'**il est produit par l'hybridome 18B6 déposé sous le numéro d'enregistrement CNCM I-3559 à la Collection Nationale de Cultures de Microorganismes (CNCM).

8. Lignée cellulaire stable produisant un anticorps selon l'une quelconque des revendications 1 à 6, **caractérisée en ce que** la souche cellulaire est choisie parmi le groupe consistant en : SP2/0, YB2/0, IR983F, le myélome humain Namalwa, PERC6, les lignées CHO, notamment CHO-K-1, CHO-Lec10, CHO-Lec1, CHO-Lec13, CHO Pro-5, CHO dhfr-, Wil-2, Jurkat, Vero, Molt-4, COS-7, 293-HEK, BHK, K6H6, NSO, SP2/0-Ag 14 et P3X63Ag8.653.

9. Hybridome 18B6 déposé sous le numéro d'enregistrement CNCM 1-3559 à la Collection Nationale de Cultures de Microorganismes (CNCM).

10. Composition pharmaceutique comprenant un anticorps selon l'une quelconque des revendications 1 à 7 et au moins un excipient et/ou au moins un véhicule pharmaceutiquement acceptables.

11. Composition selon la revendication 12, **caractérisée en ce qu'**elle comprend en outre au moins un anticorps monoclonal anti-idiotypique dirigé contre un anticorps anti-FVIII dirigé contre un domaine autre que le domaine C1 du facteur VIII, notamment un anticorps anti-idiotypique dirigé contre un anticorps anti-FVIII dirigé contre le domaine C2 du facteur VIII et/ou un anticorps dirigé contre le domaine A2 du facteur VIII.

12. Utilisation d'un anticorps selon l'une quelconque des revendications 1 à 7, pour la fabrication d'un médicament destiné au traitement de l'hémophilie de type A, notamment une hémophilie de type A avec inhibiteurs.

13. Utilisation d'un anticorps selon l'une quelconque des revendications 1 à 7, pour neutraliser *in vitro* l'activité inhibitrice d'un anticorps inhibiteur dirigé contre le domaine C1 du facteur VIII.

14. Utilisation d'un anticorps selon l'une quelconque des revendications 1 à 7, pour la détection et/ou la purification *in vitro* d'anticorps inhibiteurs du facteur VIII.

**Claims**

1. An anti-idiotypic monoclonal antibody directed against a human antibody inhibiting the factor VIII, said inhibitory antibody being directed against the C1 domain of the factor VIII, wherein the variable region of each of its light chains is coded by the nucleic acid sequence coding for the variable region of the light chain of the antibody produced by the hybridoma 18B6, deposited under the registration number CNCM 1-3559 at the "Collection Nationale de Cultures de Microorganismes' (CNCM), and the variable region of each of its heavy chains is coded by the nucleic acid sequence coding for the variable region of the heavy chain of the antibody produced by the hybridoma 18B6, deposited under the registration number CNCM I-3559 at the "Collection Nationale de Cultures de Microorganismes" (CNCM).

2. The anti-idiotypic monoclonal antibody according to claim 1, wherein the peptidic sequence of the variable region of each of its light chains is the sequence of the variable region of the light chain of the antibody produced by the hybridoma 18B6, deposited under the registration number CNCM 1-3559 at the "Collection Nationale de Cultures de Microorganismes" (CNCM), and the peptidic sequence of the variable region of each of its heavy chains is the sequence of the variable region of the heavy chain of the antibody produced by the hybridoma 18B6 deposited under the registration number CNCM 1-3559 at the "Collection Nationale de Cultures de Microorganismes" (CNCM).

3. The anti-idiotypic monoclonal antibody according to any of claims 1 to 2, **characterized in that** the inhibitory antibody is the antibody RHD5 (deposited at the BCCM/LMBP under the number LMBP 6165CB.

4. The anti-idiotypic monoclonal antibody according to any of claims 1 to 2, **characterized in that** it is a murine antibody or a chimeric antibody, notably a human hybrid antibody or a humanized antibody.

5. The anti-idiotypic monoclonal antibody according to claim 4, **characterized in that** it is a IgG1kappa.

6. The anti-idiotypic monoclonal antibody according to any of claims 1 to 5, **characterized in that** this is a fragment F(ab')2, a fragment Fab', a fragment Fab.

7. The anti-idiotypic monoclonal antibody according to any of claims 1 to 6, **characterized in that** it is produced by the hybridoma 18B6 deposited under the registration number CNCM I-3559 at the "Collection Nationale de Cultures de Microorganismes" (CNCM).

8. A stable cell line producing an antibody according to any of claims 1 to 6, **characterized in that** the cell strain is selected from the group consisting of: SP2/0, YB2/0, IR983F, human myeloma Namalwa, PERC6, CHO lines, notably CHO-K-1, CHO-Lec10, CHO-Lec1, CHO-Lec13, CHO Pro-5, CHO dhfr-, Wil-2, Jurkat, Vero, Molt-4, COS-7, 293-HEK, BHK, K6H6, NSO, Sp2/0-Ag 14 and P3X63Ag8.653.

9. The hybridoma 18B6 deposited under the registration number CNCM 1-3559 at the "Collection Nationale de Cultures de Microorganismes' (CNCM).

10. A pharmaceutical composition comprising an antibody according to any of claims 1 to 7, and at least one excipient and/or at least one carrier, both pharmaceutically acceptable.

11. The composition according to claim 12, **characterized in that** it further comprises at least one anti-idiotypic monoclonal antibody directed against an anti-FVIII antibody directed against a domain other than the domain C1 of the factor VIII, notably an anti-idiotypic antibody directed against an anti-FVIII antibody directed against the domain C2 of the factor VIII and/or an antibody directed against the domain A2 of the factor VIII.

12. The use of an antibody according to any of claims 1 to 7, for making a drug, intended for treating heamophilia of type A, notably hemophilia of type A with inhibitors.

13. The use of an antibody according to any of claims 1 to 7, for neutralizing *in vitro* the inhibitory activity of an inhibitory antibody directed against the domain C1 of the factor VIII.

14. The use of an antibody according to any of claims 1 to 7, for detecting and/or purifying *in vitro* factor VIII inhibiting antibodies.

**Patentansprüche**

1. Antiidiotypischer monoklonaler Antikörper, der gegen einen humanen Antikörper gerichtet ist, der den Faktor VIII inhibiert, wobei der inhibierende Antikörper gegen die Domäne C1 von Faktor VIII gerichtet ist, wobei die variable Region jeder seiner leichten Ketten von der Nukleinsäuresequenz kodiert wird, die die variable Region der leichten Kette des Antikörpers kodiert, der von dem Hybridom 18B6 produziert wird, das unter der Registrierungsnummer CNCM 1-3559 bei der Collection Nationale de Cultures de Microorganismes (CNCM) hinterlegt wurde, die variable Region jeder seiner schweren Ketten von der Nukleinsäuresequenz kodiert wird, die die variable Region der schweren Kette des Antikörpers kodiert, der von dem Hybridom 18B6 produziert wird, das unter der Registrierungsnummer CNCM 1-3559 bei der Collection Nationale de Cultures de Microorganismes (CNCM) hinterlegt wurde.

2. Antiidiotypischer monoklonaler Antikörper nach Anspruch 1, wobei die Peptidsequenz der variablen Region jeder seiner leichten Ketten die Sequenz der variablen Region der leichten Kette des Antikörpers ist, der von dem Hybridom 18B6 produziert wird, das unter der Registrierungsnummer CNCM 1-3559 bei der Collection Nationale de Cultures de Microorganismes (CNCM) hinterlegt wurde, und die Peptidsequenz der variablen Region jeder seiner schweren Ketten die Sequenz der variablen Region der schweren Kette des Antikörpers ist, der von dem Hybridom 18B6 produziert wird, der unter der Registrierungsnummer CNCM 1-3559 bei der Collection Nationale de Cultures de Microorganismes (CNCM) hinterlegt wurde.

3. Antiidiotypischer monoklonaler Antikörper nach einem der Ansprüche 1 bis 2, **dadurch gekennzeichnet, dass** der inhibierende Antikörper der Antikörper RHD5 ist (bei der Sammlung BCCM/LMBP unter der Nummer LMBP 6165CB hinterlegt)

4. Antiidiotypischer monoklonaler Antikörper nach einem der Ansprüche 1 bis 2, **dadurch gekennzeichnet, dass** er ein muriner Antikörper oder ein chimärer Antikörper ist, insbesondere ein hybrider humaner Antikörper oder ein humanisierter Antikörper.

5. Antiidiotypischer monoklonaler Antikörper nach Anspruch 4, **dadurch gekennzeichnet, dass** er ein IgG1kappa ist.

6. Antiidiotypischer monoklonaler Antikörper nach einem der Ansprüche 1 bis 5, **dadurch gekennzeichnet, dass** es sich um ein F(ab')2-Fragment, ein Fab'-Fragment, ein Fab-Fragment handelt.

7. Antiidiotypischer monoklonaler Antikörper nach einem der Ansprüche 1 bis 6, **dadurch gekennzeichnet, dass** er von dem Hybridom 18B6 produziert wird, das unter der Registrierungsnummer CNCM 1-3559 bei der Collection Nationale de Cultures de Microorganismes (CNCM) hinterlegt wurde.

8. Stabile Zelllinie, die einen Antikörper nach einem der Ansprüche 1 bis 6 produziert, **dadurch gekennzeichnet, dass** der Zellstamm ausgewählt ist aus der Gruppe bestehend aus: SP2/0, YB2/0, IR983F, dem humanen Myelom Namalwa, PERC6, den CHO-Linien, insbesondere CHO-K-1, CHO-Lec10, CHO-Lec1, CHO-Lec13, CHO Pro-5, CHO dhfr-, Wil-2, Jurkat, Vero, Molt-4, COS-7, 293-HEK, BHK, K6H6, NSO, SP2/0-Ag 14 und P3X63Ag8.653.

9. Hybridom 18B6, das unter der Registrierungsnummer CNCM 1-3559 bei der Collection Nationale de Cultures de Microorganismes (CNCM) hinterlegt wurde.

10. Pharmazeutische Zusammensetzung, umfassend einen Antikörper nach einem der Ansprüche 1 bis 7 und mindestens einen pharmazeutisch verträglichen Exzipienten und/oder mindestens ein pharmazeutisch verträgliches Vehikel.

**11.** Zusammensetzung nach Anspruch 12, **dadurch gekennzeichnet, dass** sie ferner mindestens einen antiidiotypischen monoklonalen Antikörper umfasst, der gegen einen Anti-FVIII-Antikörper gerichtet ist, der gegen eine Domäne gerichtet ist, die nicht die Domäne C1 von Faktor VIII ist, insbesondere einen antiidiotypischen Antikörper, der gegen einen Anti-FVIII-Antikörper gerichtet ist, der gegen die Domäne C2 von Faktor VIII gerichtet ist, und/oder einen Antikörper, der gegen die Domäne A2 von Faktor VIII gerichtet ist.

**12.** Verwendung eines Antikörpers nach einem der Ansprüche 1 bis 7 zur Herstellung eines Medikaments, das zur Behandlung von Hämophilie Typ A, insbesondere einer Hämophilie Typ A mit Inhibitoren, bestimmt ist.

**13.** Verwendung eines Antikörpers nach einem der Ansprüche 1 bis 7, um die inhibierende Aktivität eines gegen die Domäne C1 von Faktor VIII gerichteten Antikörpers *in* vitro zu neutralisieren.

**14.** Verwendung eines Antikörpers nach einem der Ansprüche 1 bis 7 zum Nachweis und/oder zur Reinigung von Faktor VIII inhibierenden Antikörpern *in vitro.*

Fig. 1

Fig. 2

Fig. 3

**Neutralisation du RHD5 par le 18B6**

Fig. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2004014955 A
- FR 0508320
- WO 2005016455 A

### Littérature non-brevet citée dans la description

- **WOOD et al.** *Nature,* 1984, vol. 312, 330-337
- **GILLES JG et al.** *Blood,* 1993, vol. 82, 2452-2461
- **JARVIS et al.** *Thromb Haemost.,* Février 1996, vol. 75 (2), 318-25
- **JACQUEMIN et al.** *Blood,* 2000, vol. 95, 156-163
- **JACQUEMIN et al.** *Blood,* 15 Juillet 1998, vol. 92 (2), 496-506
- **ANANYEVA NM et al.** *Blood Coagul Fibrinolysis,* Mars 2004, vol. 15 (2), 109-24
- **SAINT-RÉMY JM et al.** *Vox Sang,* 1999, vol. 77 (1), 21-24
- **LOLLAR et al.** *J Clin Invest.,* Juin 1994, vol. 93 (6), 2497-504
- **FAY et al.** *J Biol Chem.,* 1996, vol. 271 (11), 6027-6032
- **KABAT et al.** Sequences of Proteins of Immunological Interest. NIH Publication, 91-3242, 1991
- **JACQUEMIN et al.** *Blood,* 1998, vol. 92, 494-506
- **MORRISON et al.** *Proc. Natl. Acad. Sci. U.S.A.,* 1984, vol. 81, 6851-55
- **JONES et al.** *Nature,* 1986, vol. 321, 522
- **RIECHMANN et al.** *Nature,* 1988, vol. 332, 323
- **JACQUEMIN et al.** *Blood,* 1998, vol. 92, 496-506
- Current Protocols in Immunology. John Wiley & Sons, Inc, 1994
- **J.G. GILLES et al.** *Blood,* 2004, vol. 103, 2617-23
- **P. CORNELIS.** Les anticorps monoclonaux. *Revue IRE,* 1983, vol. 7 (4